(19) Europäisches Patentamt
European Patent Office
Office européen des brevets

(11) EP 1 037 873 B1

(12) # EUROPÄISCHE PATENTSCHRIFT

(45) Veröffentlichungstag und Bekanntmachung des Hinweises auf die Patenterteilung:
**22.08.2001 Patentblatt 2001/34**

(21) Anmeldenummer: **98961224.7**

(22) Anmeldetag: **19.11.1998**

(51) Int Cl.[7]: **C07C 239/10**

(86) Internationale Anmeldenummer:
**PCT/EP98/07445**

(87) Internationale Veröffentlichungsnummer:
**WO 99/28289 (10.06.1999 Gazette 1999/23)**

(54) **VERFAHREN ZUR HERSTELLUNG ORGANISCHER HYDROXYLAMINE**

METHOD FOR PRODUCING ORGANIC HYDROXYLAMINES

PROCEDE POUR PRODUIRE DES HYDROXYLAMINES ORGANIQUES

(84) Benannte Vertragsstaaten:
**BE DE ES FR GB NL**

(30) Priorität: **02.12.1997 DE 19753462**

(43) Veröffentlichungstag der Anmeldung:
**27.09.2000 Patentblatt 2000/39**

(73) Patentinhaber: **Consortium für elektrochemische Industrie GmbH**
**81379 München (DE)**

(72) Erfinder:
- **FREUDENREICH, Johannes**
**D-80331 München (DE)**
- **STOHRER, Jürgen**
**D-82049 Pullach (DE)**

(74) Vertreter: **Potten, Holger et al**
**Wacker-Chemie GmbH**
**Zentralabteilung Patente,**
**Marken und Lizenzen**
**Hanns-Seidel-Platz 4**
**81737 München (DE)**

(56) Entgegenhaltungen:
**EP-A- 0 147 879**
**EP-A- 0 212 375**
**DE-A- 2 327 412**
**DE-A- 2 357 370**
**DE-A- 19 502 700**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 037 873 B1

## Beschreibung

Für die Herstellung organischer Hydroxylamine sind verschiedene Verfahren beschrieben, z.B. die elektrochemische Reduktion von Nitroverbindungen oder die Reduktion von Nitroverbindungen mit Metallen wie Zink oder Amalgamen. Als großtechnische Methode wird insbesondere die selektive katalytische Hydrierung von organischen Nitroverbindungen genutzt.

Bei Nitroaromaten ist bekannt, daß die katalytische Hydrierung über mehrere Stufen verläuft. Häufig gelingt es nicht, die als Zwischenprodukte auftretenden Nitrosoaromaten und Arylhydroxylamine zu isolieren, da Weiterreduktion zum stabilen Anilinderivat erfolgt.

Darüber hinaus wird eine selektive Darstellung von Arylhydroxylaminen durch Nebenreaktionen erschwert. So disproportionieren Arylhydroxylamine zu den entsprechenden Nitrosoaromaten und Anilinderivaten. Diese Zwischenprodukte können Folgereaktionen eingehen, die zu dimeren Verbindungen wie Azoxybenzolen, Azobenzolen und Hydrazobenzolen führen.

Durch geeignete Zusätze lassen sich Disproportionierung und Überreduktion bei der Hydrierung von Nitroaromaten zurückdrängen, so daß eine selektive Darstellung möglich wird.

DE-A-2118369 (entspricht US-A-3694509; Erfinder: P. N. Rylander et al.) beschreibt eine Methode zur selektiven Darstellung von Arylhydroxylaminen, bei welcher Nitroaromaten in einem neutralen Lösungsmittel in Gegenwart von 0,1 bis 100 Mol Dimethylsulfoxid pro Mol Katalysator mit Wasserstoff hydriert werden. Die Selektivität der Hydrierung liegt unter diesen Bedingungen bei etwa 85%, so daß die erhaltenen Arylhydroxylamine noch mit großen Mengen der entsprechenden Anilinderivate verunreinigt sind und die Gesamtausbeuten der Arylhydroxylamine gering ausfallen.

In EP-A-212375 (Erfinder: G. C. Davis) werden Phosphine, Phosphate, Sulfide, Sulfoxide und heterocyclische Stickstoffverbindungen in Anwesenheit von bis zu 0,4 Äquivalenten einer Protonsäure je Mol der nitroaromatischen Verbindung als Zusatz verwendet. Im Fall der Hydrierung von p-Nitrobenzoesäureethylester lassen sich Selektivitäten bis 99% erzielen. Weitere Nitroverbindungen werden nicht beschrieben. Der notwendige Zusatz von Säuren wirkt sich jedoch negativ auf die Darstellung säureempfindlicher Arylhydroxylamine aus.

EP-A-86363 (Erfinder: D. C. Caskey, D. W. Chapman) beschreibt den Einsatz von zweiwertigen Schwefelverbindungen in Kombination mit Ammoniak, aliphatischen Aminen, Phosphin, aliphatischen Phosphinen, Arylphosphinen und Phosphitestern als Reaktionsmoderatoren zur selektiven Hydrierung von Nitroaromaten zu Arylhydroxylaminen. Die erzielbaren Selektivitäten liegen im Bereich von 89-92%. Die Inaktivierung bzw. Vergiftung der Katalysatoren insbesondere mit schwefelhaltigen Zusätzen hat zur Folge, daß die Katalysatoren meistens schon nach einem Zyklus einen Großteil ihrer Aktivität einbüßen und erneuert oder aufwendig regeneriert werden müssen.

JP 54-24837 (Erfinder: T. Tsurutani) (C.A. 91,56604) zeigt einen Prozeß auf, bei dem Arylhydroxylamine durch katalytische Hydrierung nicht substituierter oder substituierter Nitrobenzole in Alkoholen oder Ethern gewonnen werden, die Phosphorige Säure, Alkalimetallsalze der Phosphorigen Säure, Phosphorigsäureester, Thiophosphorigsäureester, Alkyl- oder Arylphosphine, Alkyl- oder Aryl- Aminophosphine, Alkyl- oder Arylsulfide, Carboalkoxyalkylsulfide, Alkyl- oder Arylmercaptane oder Thiophene enthalten. Bei diesem Verfahren muß die Hydrierung nach dem Verbrauch von zwei Äquivalenten Wasserstoff bezogen auf die Nitroverbindung abgebrochen werden, um eine Überreduktion zu den entsprechenden Anilinen zu vermeiden. Die erzielbare Selektivität liegt bei 67-98%.

Eine selektive Hydrierung von Nitrobenzolen mit Platin- oder Palladium-Katalysatoren in Anwesenheit organischer Basen, deren pK-Wert unter 3,2 beträgt, wird in DE-A-2327412 (entspricht CA-A-1001658; Erfinder: J. le Ludec) und DE-A-2357370 (entspricht GB-A-1428226; Erfinder: J. le Ludec) beschrieben. Die organische Base muß im Überschuß bezogen auf die Nitroverbindung eingesetzt werden. Bei den bevorzugten Basen Piperidin und Diethylamin lassen sich nach Aufarbeitung und Reinigung gerade bei einfach strukturierten Arylhydroxylaminen nur bescheidene Ausbeuten erzielen: N-Phenylhydroxylamin konnte nach Hydrierung von Nitrobenzol in Diethylamin als Lösungsmittel mit nur 52% isolierter Ausbeute erhalten werden (DE-A-2327412).

Weitere für diese Art der katalytischen Hydrierung von Nitroaromaten beschriebene organische Basen sind N-alkylierte und gegebenenfalls an den Kohlenstoffatomen des Ringes alkylierte Piperidine, an dem Stickstoffatom und/oder den Kohlenstoffatomen des Ringes alkylierte Pyrrolidine, N-alkylierte oder N-cycloalkylierte und gegebenenfalls an den Kohlenstoffatomen des Ringes alkylierte Aniline und Pyridin, alkylierte Pyridine, Chinolin und Isochinolin (DE-A-2455238, entspricht US-A-3927101; Erfinder: J. le Ludec) sowie für die selektive Hydrierung von Chlornitroaromaten auch sekundäre und tertiäre Monoamine, die Alkyl- oder Cycloalkylgruppen tragen (DE-A-2455887, entspricht US-A-3992395; Erfinder: J. le Ludec). Mit Pyridin als Lösungsmittel konnte N-Phenylhydroxylamin mit 83% isolierter Ausbeute gewonnen werden (DE-A-2455238).

DE-A-19502700 (entspricht ZA-A-9600610; Erfinder: N. Götz et al.) beschreibt eine Methode zur selektiven Hydrierung von Nitroaromaten mit Platinkatalysatoren oder Schwefel- bzw. Selendotierten Palladiumkatalysatoren, bei welcher Nitroaromaten in Gegenwart eines Überschusses von am Stickstoffatom substituierten Morpholinen als Reaktionsmoderatoren hydriert werden. Die beschriebenen Selektivitäten betragen bis zu 98%. Nachteilig bei diesem Verfahren ist der hohe Preis der im Überschuß verwendeten Basen. Aufgrund des hohen Siedepunktes N-alkylierter

Morpholine ist eine Entfernung dieser Basen erst bei erhöhter Temperatur möglich.

In Gegenwart von weniger als 10 Gew.% organischer Base (bezogen auf die Masse des Nitroderivats) werden Nitroaromaten und Nitroaliphaten unter Zusatz drei- oder fünfwertiger organischer Phosphorverbindungen in inerten Lösungsmitteln zu den entsprechenden Aryl- bzw. Alkylhydroxylaminen reduziert (EP-A-0147879; Erfinder: A. H. Sharma, P. Hope). Gute Ausbeuten von Arylhydroxylaminen werden vor allem beim Einsatz des kostenintensiven DMAP (N,N-Dimethylaminopyridin) im Vergleich zum Pyridin erhalten (Ausbeute N-Phenylhydroxylamin 88% mit DMAP, 76% mit Pyridin). Auch hier ist es wiederum von Nachteil, daß nur unter Einsatz von kostenintensiven, hochsiedenden und damit schwer abtrennbaren Basen hohe Selektivitäten erzielt werden. Beim Einsatz organischer Basen in Mengen von weniger als 10 Gew.% bezogen auf den Nitroaromaten werden für eine selektive Reaktion große Mengen dreiwertiger Phosphorverbindungen (0,1 - 5 Gew.%) benötigt. Dies hat eine rasche Inaktivierung des Katalysators zur Folge.

US-A-3441610 (Erfinder: J. W. Dietz, J. R. McWorther) beschreibt die katalytische Hydrierung von Nitroalkanen zu N-Alkylhydroxylaminen. Hierbei erfolgt die Hydrierung mit einem Palladium-Katalysator unter Zusatz von Eisen-, Kobalt- oder Nickel-Kationen in einem flüssigen Zweiphasensystem bestehend aus wässriger Schwefelsäure und einem nichtmischbaren organischen Solvens. Unter solchen stark aciden Bedingungen ist der Verlust an Katalysator beträchtlich. Daneben haben solche Gemische eine stark korrodierende Wirkung auf die verwendeten Apparaturen.

EP-A-0321219 (Erfinder: M.B. Sherwin, P. Pichaichanarong) beschreibt einen Prozeß zur Darstellung von unter anaeroben Bedingungen lagerstabilen wäßrigen Lösungen von Alkylhydroxylaminen durch Reduktion von Nitroaliphaten mit 1-18 Kohlenstoffatomen unter Zusatz von Komplexbildnern wie EDTA. Die Lagerstabilität solcher Lösungen wird durch die Verringerung anwesender Übergangsmetallionen auf weniger als 20 ppm erzielt.

Der Erfindung liegt die Aufgabe zugrunde, ein Verfahren zur Herstellung von organischen Hydroxylaminen bereitzustellen, das die im Stand der Technik genannten Nachteile vermeidet, großtechnisch einfach und kostengünstig durchführbar ist, hohe Selektivitäten und Ausbeuten aufweist und eine schonende Isolierung der organischen Hydroxylamine ermöglicht.

Die Aufgabe wird gelöst durch ein Verfahren, bei dem mindestens eine organische Nitroverbindung in Anwesenheit einer organischen Base oder Ammoniaks und einer dreiwertigen Phosphorverbindung und eines Hydrierkatalysators partiell hydriert wird, dadurch gekennzeichnet, daß die organische Base oder der Ammoniak in Mengen größer 10 Gew.% bezogen auf die organische Nitroverbindung eingesetzt werden.

Die Kombination von organischer Base oder Ammoniak in Anteilen von über 10 Gew.% bezogen auf die Nitroverbindung mit dreiwertigen Phosphorverbindungen hat im Vergleich zur alleinigen Verwendung überschüssiger organischer Basen (DE-A-2327412, DE-A-2357370, DE-A-2455238, DE-A-2455887, DE-A-19502700) eine signifikante Steigerung der Selektivität zur Folge.

Organische Base und Ammoniak werden im folgenden unter dem Begriff Stickstoffbase zusammengefaßt.

Beim erfindungsgemäßen Verfahren ist die Selektivität der Reaktion unabhängig von der Art der verwendeten Stickstoffbase. Das Verfahren ermöglicht damit die Verwendung kostengünstiger und gut verfügbarer Stickstoffbasen.

Gerade bei Verwendung der kostengünstigen offenkettigen primären, sekundären und tertiären Amine sind im Temperaturbereich über 20°C nur unter Zusatz dreiwertiger Phosphorverbindungen gute Selektivitäten zu erzielen, was aufgrund der Exothermie der Hydrierung große Vorteile für die Prozeßtechnik hat.

Die Wahl tiefsiedender Stickstoffbasen wie primäre Amine oder Ammoniak macht eine leichte Abtrennung der Base auch von sehr thermolabilen organischen Hydroxylaminen möglich.

Da eine Weiterreduktion der gebildeten organischen Hydroxylamine zu den entsprechenden Aminderivaten beim Einsatz des erfindungsgemäßen Verfahrens sehr stark verlangsamt ist, erfolgt nach Aufnahme von zwei Molâquivalenten Wasserstoff bezogen auf die Nitroverbindung bis zu mehreren Stunden keine weitere Wasserstoffaufnahme. Das erfindungsgemäße Verfahren erleichtert damit auch die Bestimmung des Endpunktes der Hydrierung und vereinfacht die Implementierung in industrielle Anlagen.

Die nach dem erfindungsgemäßen Verfahren erhaltenen Lösungen der Alkylhydroxylamine sind unter Schutzgas lagerstabil.

Die vorliegende Erfindung betrifft vorzugsweise ein Verfahren zur Herstellung von organischen Hydroxylaminen der allgemeinen Formel **I** oder **II**

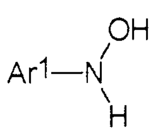

**I**

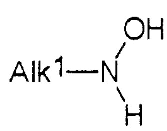

**II**

bei welchen $Ar^1$ ein unsubstituierter oder substituierter isocyclischer oder heterocyclischer Arylrest ist und $Alk^1$ ein unsubstituierter oder substituierter Alkylrest ist, durch partielle Hydrierung von organischen Nitroverbindungen der allgemeinen Formel **III** oder **IV**

$Ar^1—NO_2$ $Alk^1—NO_2$

**III** **IV**

bei denen $Ar^1$ und $Alk^1$ die genannten Bedeutungen haben, in Gegenwart eines Hydrierkatalysators enthaltend mindestens ein Element der 7. oder 8. Nebengruppe des Periodensystems und mindestens eine Stickstoffbase und mindestens eine dreiwertige Phosphorverbindung, dadurch gekennzeichnet, daß die Stickstoffbase und die Nitroverbindung in einem Gewichtsverhältnis von Stickstoffbase zu Nitroverbindung größer 0,1 eingesetzt werden und die dreiwertige Phosphorverbindung in einer Menge von 0,0001 - 3 Mol% bezogen auf die Nitroverbindung eingesetzt wird.

$Ar^1$ steht in den Formeln **I** und **III** vorzugsweise für einen ein- oder zweikernigen iso- oder heterocyclischen Arylrest, der ein- oder mehrfach mit $R^1$ substituiert sein kann, wobei $R^1$ gleich oder verschieden ist und eine der folgenden Bedeutungen hat:
Halogenatom, $C_1$-$C_4$-Alkyl, Halogen- ($C_1$-$C_4$)-Alkyl, Halogen($C_1$-$C_4$)-Alkoxy, $C_1$-$C_4$-Alkoxy,
$C_1$-$C_4$-Alkylthio, ($C_1$-$C_4$)-Alkylcarbonyl, ($C_1$-$C_4$)-Alkoxycarbonyl, ($C_1$-$C_4$)-Alkylaminocarbonyl, ($C_1$-$C_4$)-Dialkylaminocarbonyl, ($C_1$-$C_4$)-Dialkylamino, ($C_1$-$C_4$)-Alkylamino,
($C_1$-$C_4$)-Alkylcarbonylamino,
($C_1$-$C_4$)-Alkylcarbonyl- ($C_1$-$C_4$)-alkylamino, Cyano, Nitro, Amino, Hydroxy, Carboxy, Ester oder Salz des Carboxyrest, Sulfono, Ester oder Salz des Sulfonorests oder A-B,
wobei A -O-, -S-, -O-$CH_2$-, -$CH_2$-O-, -S-$CH_2$-, -$CH_2$-S-, -$CH_2$-O-CO-, -$CH_2$-N($R^2$)-, -CH=CH-, -CH=N-O- oder eine Einfachbindung bedeutet, und
$R^2$ $C_1$-$C_4$-Alkyl, $C_2$-$C_4$-Alkenyl oder Wasserstoff bedeutet und
B Phenyl, Naphthyl, Pyridinyl, Pyrazinyl, Pyrimidinyl, Pyridazinyl, Pyrazolyl, Imidazolyl, Oxazolyl, Isoxazolyl, Thiazolyl, Isothiazolyl, Furanyl, Thienyl, Pyrrolyl oder $C_3$-$C_7$-Cycloalkyl bedeutet,
wobei B mit 1-3 Substituenten $R^3$ substituiert sein kann und
$R^3$ Halogen, $C_1$-$C_4$-Alkyl, Hab-($C_1$-$C_4$)-Alkyl, Halo- ($C_1$-$C_4$)-Alkoxy,
$C_1$-$C_4$-Alkoxy, $C_1$-$C_4$-Alkylthio, ($C_1$-$C_4$)-Alkylcarbonyl,
($C_1$-$C_4$)-Alkoxycarbonyl, ($C_1$-$C_4$)-Alkylaminocarbonyl,
($C_1$-$C_4$)-Dialkylaminocarbonyl, ($C_1$-$C_4$)-Alkylcarbonylamino,
($C_1$-$C_4$)-Alkylcarbonyl-($C_1$-$C_4$)-alkylamino oder Cyano bedeutet.

$Alk^1$ steht in den Formeln **II** und **IV** vorzugsweise für einen linearen oder verzweigten oder cyclischen Alkylrest enthaltend 1 - 24 C-Atome, wobei der Alkylrest gesättigt oder ungesättigt sein kann und nicht substituiert oder ein- oder mehrfach mit dem Substituenten $R^4$ substituiert ist, wobei $R^4$ gleich oder verschieden ist und eine der folgenden Bedeutungen hat:
$C_1$-$C_4$-Alkoxy, $C_1$-$C_4$-Alkylthio, ($C_1$-$C_4$)-Alkylcarbonyl,
($C_1$-$C_4$)-Alkoxycarbonyl, ($C_1$-$C_4$)-Alkylaminocarbonyl,
($C_1$-$C_4$)-Dialkylaminocarbonyl, ($C_1$-$C_4$)-Dialkylamino,
($C_1$-$C_4$)-Alkylamino, ($C_1$-$C_4$)-Alkylcarbonylamino,
($C_1$-$C_4$)-Alkylcarbonyl- ($C_1$-$C_4$)-alkylamino, Amino, Hydroxy.

Besonders bevorzugt ist das Verfahren zur Darstellung von N-Phenylhydroxylamin geeignet.

Als organische Nitroverbindungen lassen sich im erfindungsgemäßen Verfahren beispielsweise sowohl einfache Nitroaromaten wie Nitrobenzol, 2-Nitrotoluol, 4-Nitrotoluol, 4-Nitrocumol, 2-Nitro-m-xylol, 3-Nitro-o-xylol, 4-Nitro-o-xylol, 4-Nitro-m-xylol, 5-Nitro-m-xylol, Nitromesitylen,Nitroanthracen, 1-Nitronaphthalin, 2-Nitronaphthalin, 1-Nitropyren, 2-Methyl-1-nitronaphthalin, 2-Nitrofluoren, als auch komplexere Verbindungen wie 2-Nitroanisol, 3-Nitroanisol, 4-Nitroanisol, 4-Nitrobiphenyl, 2-Nitrobenzolsulfonsäureamid, 5-Nitrochinolin, 8-Nitrochinolin, 2-Nitroimidazol, 5-Nitrindazol, 6-Nitroindolin, 5-Nitroisochinolin, 4-Nitro-α-naphtholmethylether, 4-Nitrophenol, 4-Nitroanilin, Nitroresorcin, 2-Nitropyridin, 4-Nitrobenzoesäureamid, 4-Nitrobenzonitril, 4-Nitro-2,1,3-benzothiazol einsetzen als auch Nitroaliphaten wie beispielsweise Nitromethan, Nitro-tert.-butan, 1-Nitrobutan, 3-Nitro-2-butanol, Nitrocyclohexan, 1-Nitrocyclohexen, Nitrocyclopentan, Nitroethan, 2-Nitroethanol, 1-Nitrohexan, 1-Nitropropan, 2-Nitropropan, 2-Nitro-1-propanol, 2-Nitro-1-propylamin, 2-Nitro-1-ethylamin.

Von den Nitroaromaten bevorzugt sind Nitrobenzol, 2-Nitrotoluol, 4-Nitrotoluol, 4-Nitrocumol, 2-Nitro-m-xylol, 3-Nitro-o-xylol, 4-Nitro-o-xylol, 4-Nitro-m-xylol, 5-Nitro-m-xylol, Nitromesitylen,Nitroanthracen, 1-Nitronaphthalin, 2-Nitronaphthalin.

Von den Nitroaliphaten bevorzugt sind Nitromethan, Nitro-tert.-butan, 1-Nitrobutan, Nitrocyclohexan, 1-Nitropropan, 2-Nitropropan.

Die als Edukte verwendeten Nitroaromaten und Nitroaliphaten sind käuflich erhältlich oder in bekannter Art auf einfachem Weg und mit guten Ausbeuten darstellbar (R.G. Coombes in Comprehensive Organic Chemistry, Pergamon Press, Oxford-New YorkToronto-Sydney-Paris-Frankfurt, Vol. 2, Part 7, 325-334, 1979)

Der im erfindungsgemäßen Verfahren verwendete Hydrierkatalysator kann gegebenenfalls mit Silber, Gold, Kupfer, Zinn, Schwefel, Selen oder Kombinationen dieser Elemente dotiert sein.

Der Hydrierkatalysator kann vorzugsweise auf einem Trägermaterial aufgebracht eingesetzt werden.

Geeignete Trägermaterialien sind vorzugsweise ausgewählt aus der Gruppe poröse oder nichtporöse Kohlenstoffsorten mit geringen oder großen spezifischen Oberflächen, oxidische oder salzartige Verbindungen wie z.B. Aluminiumoxide, Silikate, Calciumcarbonat, Bariumcarbonat, Bariumsulfat, Titandioxid und Kombinationen dieser Materialien.

Darüber hinaus kann der Katalysator auch auf Lösungsmittelinerten Metallen wie Eisen, Kupfer, Nickel, Blei, Tantal, Titan, Molybdän, Chrom, Vanadium oder Legierungen der genannten Verbindungen, gegebenenfalls dotiert mit Kohlenstoff, aufgebracht sein.

Als Katalysator bevorzugt sind Platin, Palladium und Nickel.

Als Katalysator besonders bevorzugt sind Platin und Palladium.

Als Trägermaterial bevorzugt sind poröse oder nichtporöse Kohlenstoffsorten, Kombinationen von porösen oder nichtporösen Kohlenstoffsorten mit oxidischen oder salzartigen Verbindungen und die in den Lösungsmitteln inerten Metalle Eisen, Kupfer, Titan, Tantal, Vanadium und deren, gegebenenfalls mit Kohlenstoff dotierte, Legierungen.

Liegt der Katalysator auf einem Trägermaterial gebunden vor, dann beträgt der Katalysatorgehalt vorzugsweise 0,1 - 20 Gew.%, besonders bevorzugt 0,1 - 10 Gew.% und insbesondere bevorzugt 0,1 - 4 Gew.%. bezogen auf das Trägermaterial.

Die Menge des eingesetzten Katalysator-Metalls beträgt vorzugsweise zwischen 0,001 und 1 Gew.%, besonders bevorzugt zwischen 0,001 und 0,1 Gew.% bezogen auf die eingesetzte Nitroverbindung.

Als Stickstoffbase wird im erfindungsgemäßen Verfahren vorzugsweise eine Verbindung ausgewählt aus der Gruppe Ammoniak, Monoalkylamine, Dialkylamine, Trialkylamine, Monoalkanolamine, Dialkanolamine, Trialkanolamine, (Poly)alkylenpolyamine, N-alkylierte und gegebenenfalls an den Kohlenstoffatomen des Ringes alkylierte Piperidine und Morpholine, an dem Stickstoffatom und/oder den Kohlenstoffatomen des Ringes alkylierte Pyrrolidine, N-alkylierte und gegebenenfalls an den Kohlenstoffatomen des Ringes alkylierte Aniline und Pyridin, alkylierte Pyridine, Chinolin und Isochinolin eingesetzt. Die genannten Alkyl- und Alkanolgruppen können verzweigt oder unverzweigt sein und besitzen bevorzugt 1 - 20 C-Atome, besonders bevorzugt 1 - 6 C-Atome und insbesondere bevorzugt 1 - 4 C-Atome.

Beispiele für Mono-, Di- und Trialkylamine sind Methylamin, Ethylamin, 1-Propylamin, 2-Propylamin, Pentylamin, Hexylamin, Heptylamin, Octylamin, Nonylamin, Dimethylamin, Diethylamin, Dipropylamin, Diisopropylamin, Dipentylamin, Dihexylamin, Diheptylamin, Dioctylamin, Trimethylamin, Triethylamin, Tripropylamin, Ethyldimethylamin, Diethylmethylamin, Triisopropylamin, Tributylamin, Tripentylamin, Trihexylamin, Triheptylamin, Trioctylamin, Cyclohexylamin.

Beispiele für Mono-, Di- und Trialkanolamine sind Ethanolamin, Propanolamin, Butanolamin, Diethanolamin und Triethanolamin.

Beispiele für (Poly)alkylenpolyamine sind Ethylendiamin, Diethylentriamin, Triethylentetramin, Tetraethylenpentamin.

Beispiele für Pyrrolidine, Piperidine und Morpholine sind Pyrrolidin, Piperidin, Morpholin und Mono-, Di-, Tri- und Tetramethyl- Pyrrolidine, -Piperidine und -Morpholine, N-Methylpiperidin, N-Methylmorpholin, N-Methylpyrrolidin, N-Ethylpiperidin, N-Ethylmorpholin, N-Ethylpyrrolidin.

Beispiele für Aniline und Pyridine sind Anilin, o- und m-Toluidin, 2-Ethylanilin, 3-Ethylanilin, 4-Ethylanilin, Pyridin, 2-Picolin, 3-Picolin, 4-Picolin, 2-Ethylpyridin.

Bevorzugt wird eine Verbindung ausgewählt aus der Gruppe Ammoniak, Mono-, Di- und Trialkylamine, Morpholine und Piperidine eingesetzt.

Besonders bevorzugt wird eine Verbindung ausgewählt aus der Gruppe Ammoniak und Mono-, Di- und Trialkylamine wie Methylamin, Ethylamin, 1-Propylamin, 2-Propylamin, Pentylamin, Hexylamin, Heptylamin, Octylamin, Nonylamin, Dimethylamin, Diethylamin, Dipropylamin, Diisopropylamin, Dipentylamin, Dihexylamin, Diheptylamin, Dioctylamin, Trimethylamin, Triethylamin, Tripropylamin, Ethyldimethylamin, Diethylmethylamin, Triisopropylamin, Tributylamin, Tripentylamin, Trihexylamin, Triheptylamin, Trioctylamin, Cyclohexylamin eingesetzt.

Die Stickstoffbasen können einzeln oder als Gemische eingesetzt werden, wobei die eingesetzte Menge an Base derart sein muß, daß das Gewichtsverhältnis eingesetzte Stickstoffbase/ eingesetzte Nitroverbindung größer 0,1 ist. Vorzugsweise beträgt das Gewichtsverhältnis zwischen 0,1 und 10.

Für das erfindungsgemäße Verfahren sind vorzugsweise solche dreiwertige (trivalente) Phosphorverbindungen geeignet, bei denen das Phosphoratom eine oder mehrere Aryl- oder Aryloxygruppen und/oder Alkyl- oder Alkyloxygruppen trägt, deren Kettenlänge 1-20 Kohlenstoffatome beträgt, wobei die Arylgruppen unsubstituiert oder mit $C_1$-$C_4$-Alkyl- oder $C_1$-$C_4$-Alkoxygruppen substituiert sein können.

Beispiele für solche Verbindungen sind Triphenylphosphit, Dimethylphenylphosphit, Triphenylphosphin, Diphenylphosphit, Dip-nitrophenylphosphit und Di-p-methylphenylphosphit, Trimethylphosphin, Triethylphosphin, Tripropylphosphin, Triisopropylphosphin, Tributylphosphin, Trioctylphosphin, Trimethylphosphit, Triethylphosphit, Triisopropylphosphit, Tripropylphosphit, Tributylphosphit, Trihexylphosphit, Trilaurylphosphit, Tridecylphosphit, Trinonylphosphit.

Bevorzugt sind Phosphorverbindungen, bei denen das Phosphoratom eine oder mehrere Phenyl- oder Phenyloxygruppen und/oder eine oder mehrere Alkyl- oder Alkyloxygruppen trägt, deren Kettenlänge 1-8 Kohlenstoffatome beträgt.

Weitere dreiwertige Phosphorverbindungen, die im erfindungsgemäßen Verfahren verwendet werden können, sind Phosphortrichlorid und Phosphorige Säure.

Die dreiwertigen Phosphorverbindungen können einzeln oder als Gemische eingesetzt werden.

Vorzugsweise sollte die dreiwertige Phosphorverbindung in der Startreaktionsmischung in Mengen von 0,0001 - 3 Mol% bezogen auf die Nitroverbindung vorhanden sein, bevorzugt in Mengen von 0,0001 - 0,8 Mol% und am meisten bevorzugt in Mengen von 0,0001 - 0,1 Mol%.

Das erfindungsgemäße Verfahren wird vorzugsweise im Temperaturbereich zwischen -10°C und 100°C, bevorzugt zwischen 10°C und 80°C durchgeführt.

Um zu gewährleisten, daß die Hydrierung bei der gewählten Temperatur ausreichend schnell verläuft, wird vorzugsweise ein Wasserstoff-Partialdruck eingestellt, der zwischen Normaldruck und 10 bar Überdruck liegt.

Die Reaktion kann in Anwesenheit weiterer Lösungsmittel wie Ether, Ester, Alkohole, Nitrile, Amide, aliphatische oder aromatische Kohlenwasserstoffe oder Wasser durchgeführt werden.

Die nach dem erfindungsgemäßen Verfahren gewonnenen Arylhydroxylamine können, wie literaturbekannt, über die Bamberger-Umlagerung in präparativ wertvolle substituierte Aminophenole überführt werden.

Arylhydroxylamine finden bekanntermaßen weiterhin Anwendung als Ausgangsmaterialien für Diarylnitrone, die in der Photolithographie eingesetzt werden, als Zwischenprodukte für die Herstellung von Pflanzenschutzmitteln und als Ausgangsmaterial zur Darstellung von Cupferron, einem Analysenreagens.

Auch die nach dem erfindungsgemäßen Verfahren gewonnenen Alkylhydroxylamine finden insbesondere als Zwischenprodukte für die Darstellung von Hydroxamsäuren, die in der Pharmazie und im Pflanzenschutz eingesetzt werden, vielfältige Anwendung.

Die folgenden Beispiele dienen der Veranschaulichung der vorliegenden Erfindung.

**Beispiel 1**

In einen 100 ml-Autoklaven gibt man 68 mg eines Katalysators, der 5 Gew.-% Platin auf Aktivkohle (Fa. Aldrich) enthält. Dazu pipettiert man 3,0 ml Nitrobenzol und 12,0 ml Diethylamin. Nach Zusatz von 3 µl Triethylphosphit wird das Gemisch bei 25°C unter starkem Rühren bei einem Wasserstoffdruck von 5 bar 2 Stunden hydriert.
Die HPLC-Analyse des Rohgemisches ergibt 96,6 Mol% Phenyl-hydroxylamin, 1,4 Mol% Anilin und 2 Mol% Nitrobenzol.

**Vergleichsbeispiel 1**

Es wird analog Beispiel 1 verfahren, aber kein Triethylphosphit zugesetzt.
Die HPLC-Analyse nach 2 h Reaktionszeit ergibt 46,2 Mol% Phenylhydroxylamin, 47,2 Mol% Anilin und 6,6 Mol% Nitrobenzol.

**Beispiel 2**

Es wird analog Beispiel 1 verfahren. Als Lösungsmittel wird Triethylamin eingesetzt. Der Katalysator wird aus einer vorhergehenden Hydrierreaktion wieder eingesetzt. Zur vollständigen Reaktion werden 1,5 h benötigt.
Die HPLC-Analyse ergibt 97,3 Mol% Phenylhydroxylamin, 1,0 Mol% Anilin und 1,7 Mol% Nitrobenzol.

**Vergleichsbeispiel 2**

Es wird analog Beispiel 2 gearbeitet, aber kein Triethylphosphit zugesetzt. Die Hydrierung wird innerhalb 2 h bei 20°C durchgeführt.
Die HPLC-Analyse ergibt 62,9 Mol% Phenylhydroxylamin, 16,2 Mol% Nitrobenzol und 20,9 Mol% Anilin.

**Beispiel 3**

Es wird analog Beispiel 1 verfahren. Als Lösungsmittel wird Pyridin eingesetzt.
Die HPLC-Analyse ergibt nach 3,75 h Reaktionszeit 97,3 Mol% Phenylhydroxylamin, 2,4 Mol% Anilin und 0,3 Mol% Nitrobenzol.

**Vergleichsbeispiel 3**

Es wird analog Beispiel 3 verfahren, aber kein Triethylphosphit zugesetzt. Die Reaktionszeit beträgt 4 h.
Die HPLC-Analyse ergibt 91,2 Mol% Phenylhydroxylamin, 6,5 Mol% Anilin und 2,3 Mol% Nitrobenzol

**Beispiel 4**

Es wird analog Beispiel 1 verfahren. Als Lösungsmittel wird N-Methylmorpholin eingesetzt. Die Hydrierung wird innerhalb 5 h bei 28°C durchgeführt.
Die HPLC-Analyse ergibt 97,8 Mol% Phenylhydroxylamin, 1,7 Mol% Anilin und 0,5 Mol% Nitrobenzol.

**Vergleichsbeispiel 4**

Es wird analog Beispiel 4 gearbeitet, aber kein Triethylphosphit zugesetzt.
Die HPLC-Analyse ergibt nach 5 h Reaktionszeit 85 Mol% Phenyl-hydroxylamin, 14,6 Mol% Anilin und 0,4 Mol% Nitrobenzol.

**Beispiel 5**

In einen 100-ml-Autoklaven gibt man 45,9 mg eines Katalysators, der 1,2 Gew.-% Platin auf Aktivkohle enthält. Dazu pipettiert man 3,6 g Nitrobenzol und 12 ml n-Propylamin. Nach Zusatz von 1 µl Triethylphosphit wird das Gemisch bei 25°C unter Rühren bei 2 bar Wasserstoffdruck hydriert.
Nach einer Reaktionszeit von 3,75 h ergibt die HPLC-Analyse 98,2 Mol% Phenyl-hydroxylamin und 1,8 Mol% Anilin.

**Vergleichsbeispiel 5**

Es wird analog Beispiel 5 verfahren und kein Triethylphosphit zugesetzt.
Die HPLC-Analyse ergab nach einer Reaktionszeit von 4,25 h 82,6 Mol% Phenylhydroxylamin, 16,5 Mol% Anilin und 0,9 Mol% Nitrobenzol.

**Beispiel 6**

Es wird analog Beispiel 5 gearbeitet. Als Lösungsmittel wird i-Propylamin eingesetzt. Dazu gibt man einen Katalysator, der 5 Gew.-% Platin auf Aktivkohle (Fa. Aldrich) enthält. Zur vollständigen Reaktion werden 2,5 h benötigt.
Die HPLC-Analyse ergab 97,7 Mol% Phenylhydroxylamin, 2,0 Mol% Anilin und 0,3 Mol% Nitrobenzol.

**Beispiel 7**

Es wird analog Beispiel 5 verfahren. Als Lösungsmittel wird ein Gemisch von 6 ml n-Propylamin und 6 ml Toluol eingesetzt. Es werden 68 mg eines Katalysators eingesetzt, der 5 Gew.-% Platin auf Aktivkohle enthält (Fa. Aldrich). Zur vollständigen Hydrierung werden 3h benötigt.
Die HPLC-Analyse ergab 93,2 Mol% Phenylhydroxylamin, 3,9 Mol% Anilin und 2,9 Mol% Nitrobenzol.

**Beispiel 8**

Es wird analog Beispiel 5 verfahren. Es werden 68 mg eines Katalysators eingesetzt, der 5 Gew.-% Platin auf Aktivkohle enthält (Fa. Aldrich). Das Triethylphosphit wird durch 4,6 mg Triphenylphosphin ersetzt. Zur vollständigen Umsetzung wird 3,75 h begast.
Die HPLC-Analyse ergibt 96,6 Mol% Phenylhydroxylamin, 2,0 Mol% Anilin und 1,4 Mol% Nitrobenzol.

**Beispiel 9**

Es wird analog Beispiel 8 verfahren und das Triphenylphosphin durch 3,5 µl Tripropylphosphin ersetzt.
Die HPLC-Analyse ergibt nach 2,75 h Reaktionszeit 96,5 Mol% Phenylhydroxylamin, 1,6 Mol% Anilin und 1,9 Mol% Nitrobenzol.

**Beispiel 10**

Es wird analog Beispiel 5 gearbeitet. Es werden 22,7 mg eines Katalysators eingesetzt, der 5 Gew.-% Platin auf Aktivkohle enthält (Fa. Aldrich). Die Hydrierreaktion wird bei 60°C durchgeführt. Nach einer Hydrierungszeit von 2,5 h erhält man nach HPLC-Analyse 95,3 Mol% Phenylhydroxylamin, 2,0 Mol% Anilin und 2,7 Mol% Nitrobenzol.

**Beispiel 11**

In einen 100-ml-Autoklaven gibt man 31,5 mg Platinpulver (Fa. Alfa 99,9 % Korngröße 0,5-1,2 µ) Dazu pipettiert man 3,6 g Nitrobenzol und 12 ml n-Propylamin. Nach Zusatz von 3 µl Triethylphosphit wird das Gemisch bei 30°C unter Rühren bei 5 bar Wasserstoffdruck hydriert.
Die HPLC-Analyse ergab nach 2 h Reaktionszeit 95,4 Mol% Phenylhydroxylamin 2,0 Mol% Anilin und 3,6 Mol% Nitrobenzol.

**Beispiel 12**

In einen 100 ml-Autoklaven gibt man 110 mg eines Katalysators, der 1 % Platin auf Aktivkohle enthält. Dazu gibt man 5,09 g 8-Nitrochinolin und 20 ml n-Propylamin. Nach Zusatz von 3 µl Triethylphosphit wird das Gemisch bei 25 °C unter starkem Rühren bei einem Wasserstoffdruck von 5 bar 3 Stunden hydriert. Die HPLC-Analyse ergibt 100 Mol% 8-Hydroxylaminochinolin.
Das Rohgemisch wird bei 40 °C / 20 mbar vom Lösungsmittel befreit, in Ethylacetat gelöst und mit Petrolether ausgefällt. Man erhält ein gelbes Pulver mit Schmp. 66-68 °C.
1H-NMR ($CD_3OD$ δ in ppm) : 8.71 (dd, 1 H), 8.19 (dd, 1 H), 7,42 (m, 4 H).

**Beispiel 13**

In einen 100 ml-Autoklaven gibt man 92 mg eines Katalysators, der 1,2% Platin auf Aktivkohle enthält. Dazu pipettiert man 15 ml eines Gemisches aus 9,6 ml Nitrobenzol, 38,5 ml n-Hexanol und 1,9 ml Wasser. In dieses Gemisch wird 4,0 g gasförmiger Ammoniak eingeleitet. Nach Zusatz von 3 µl Triethylphosphit wird das Gemisch bei Raumtemperatur und 5 bar Wasserstoffpartialdruck 3,25 h hydriert.
Die HPLC-Analyse ergibt 95,25 Mol% Phenylhydroxylamin, 3 Mol% Anilin und 1,75 Mol% Nitrobenzol.

**Beispiel 14**

In einen 100 ml-Autoklaven gibt man 110 mg eines Katalysators, der 1% Platin auf Aktivkohle enthält. Dazu pipettiert man 15 ml eines Gemisches aus 18,7 ml Nitrobenzol, 74,8 ml n-Butanol und 6,6 ml Wasser. In dieses Gemisch wird 7,9 g gasförmiger Ammoniak eingeleitet. Nach Zusatz von 3 µl Triethylphosphit wird das Gemisch bei Raumtemperatur und 5 bar Wasserstoffpartialdruck 2,25 h hydriert.
Die HPLC-Analyse ergibt 96 Mol% Phenylhydroxylamin, 1,5 Mol% Nitrobenzol und 2,5 Mol% Anilin.

**Beispiel 15**

In einen 100 ml-Autoklaven gibt man 110 mg eines Katalysators, der 1% Platin auf Aktivkohle enthält. Dazu pipettiert man 3 ml Nitrobenzol und 12 ml einer 7 N Ammoniaklösung in Methanol. Nach Zusatz von 3 µl Triethylphosphit wird bei Raumtemperatur und 5 bar Wasserstoffpartialdruck 5,25 h hydriert.
Die HPLC-Analyse ergibt 95 Mol% Phenylhydroxylamin, 3,3 Mol% Anilin und 0,7 Mol% Nitrobenzol.

**Patentansprüche**

1. Verfahren zur Herstellung von organischen Hydroxylaminen bei dem eine organische Nitroverbindung in Anwesenheit einer organischen Base oder Ammoniaks, einer dreiwertigen Phosphorverbindung und eines Hydrierkatalysators partiell hydriert wird, dadurch gekennzeichnet, daß die organische Base in Mengen größer 10 Gew.% bezogen auf die organische Nitroverbindung eingesetzt wird.

2. Verfahren zur Herstellung von organischen Hydroxylaminen der allgemeinen Formel **I** oder **II**

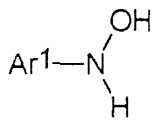

I

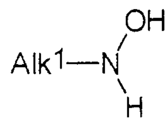

II

bei welchen $Ar^1$ ein unsubstituierter oder substituierter isocyclischer oder heterocyclischer Arylrest ist und $Alk^1$ ein unsubstituierter oder substituierter Alkylrest ist, durch partielle Hydrierung von organischen Nitroverbindungen der allgemeinen Formel **III** oder **IV**

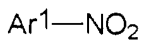

III

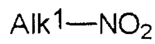

IV

bei denen $Ar^1$ und $Alk^1$ die genannten Bedeutungen haben, in Gegenwart eines Hydrierkatalysators enthaltend mindestens ein Element der 7. oder 8. Nebengruppe des Periodensystems, und mindestens eine Stickstoffbase und mindestens eine dreiwertige Phosphorverbindung, dadurch gekennzeichnet, daß die Stickstoffbase und die Nitroverbindung in einem Gewichtsverhältnis von organischer Base zu Nitroverbindung größer 0,1 eingesetzt werden und die dreiwertige Phosphorverbindung in einer Menge von 0,0001 - 3 Mol% bezogen auf die Nitroverbindung eingesetzt wird.

3. Verfahren nach Anspruch 2, dadurch gekennzeichnet, daß $Ar^1$ in den Formeln **I** und **III** steht für einen ein- oder zweikernigen iso- oder heterocyclischen Arylrest, der ein- oder mehrfach mit $R^1$ substituiert sein kann, wobei $R^1$ gleich oder verschieden ist und eine der folgenden Bedeutungen hat: Halogenatom, $C_1$-$C_4$-Alkyl, Halogen-($C_1$-$C_4$)-Alkyl, Halogen-($C_1$-$C_4$)-Alkoxy, $C_1$-$C_4$-Alkoxy, $C_1$-$C_4$-Alkylthio, ($C_1$-$C_4$)-Alkylcarbonyl, ($C_1$-$C_4$)-Alkoxycarbonyl, ($C_1$-$C_4$)-Alkylaminocarbonyl, ($C_1$-$C_4$)-Dialkylaminocarbonyl, ($C_1$-$C_4$)-Dialkylamino, ($C_1$-$C_4$)-Alkylamino, ($C_1$-$C_4$)-Alkylcarbonylamino, ($C_1$-$C_4$)-Alkylcarbonyl- ($C_1$-$C_4$)-alkylamino, Cyano, Nitro, Amino, Hydroxy, Carboxy, Ester oder Salz des Carboxyrest, Sulfono, Ester oder Salz des Sulfonorests oder A-B,
wobei A -O-, -S-, -O-$CH_2$-, -$CH_2$-O-, -S-$CH_2$-, -$CH_2$-S-, -$CH_2$-O-CO-, -$CH_2$-N($R^2$)-, -CH=CH-, -CH=N-O- oder eine Einfachbindung bedeutet, und
$R^2$ $C_1$-$C_4$-Alkyl, $C_2$-$C_4$-Alkenyl oder Wasserstoff bedeutet und
B Phenyl, Naphthyl, Pyridinyl, Pyrazinyl, Pyrimidinyl, Pyridazinyl, Pyrazolyl, Imidazolyl, Oxazolyl, Isoxazolyl, Thiazolyl, Isothiazolyl, Furanyl, Thienyl, Pyrrolyl oder $C_3$-$C_7$-Cycloalkyl bedeutet,
wobei B mit 1-3 Substituenten $R^3$ substituiert sein kann und $R^3$ Halogen, $C_1$-$C_4$-Alkyl, Halo-($C_1$-$C_4$)-Alkyl, Halo-($C_1$-$C_4$)-Alkoxy,
$C_1$-$C_4$-Alkoxy, $C_1$-$C_4$-Alkylthio, ($C_1$-$C_4$)-Alkylcarbonyl,
($C_1$-$C_4$)-Alkoxycarbonyl, ($C_1$-$C_4$)-Alkylaminocarbonyl,
($C_1$-$C_4$)-Dialkylaminocarbonyl, ($C_1$-$C_4$)-Alkylcarbonylamino,
($C_1$-$C_4$)-Alkylcarbonyl-($C_1$-$C_4$)-alkylamino oder Cyano bedeutet, und $Alk^1$ in den Formeln **II** und **IV** steht für einen linearen oder verzweigten oder cyclischen Alkylrest enthaltend 1 - 24 C-Atome, wobei der Alkylrest gesättigt oder ungesättigt sein kann und nicht substituiert oder ein- oder mehrfach mit dem Substituenten $R^4$ substituiert ist, wobei $R^4$ gleich oder verschieden ist und eine der folgenden Bedeutungen hat:
$C_1$-$C_4$-Alkoxy, $C_1$-$C_4$-Alkylthio, ($C_1$-$C_4$)-Alkylcarbonyl,
($C_1$-$C_4$)-Alkoxycarbonyl, ($C_1$-$C_4$)-Alkylaminocarbonyl,
($C_1$-$C_4$)-Dialkylaminocarbonyl, ($C_1$-$C_4$)-Dialkylamino,
($C_1$-$C_4$)-Alkylamino, ($C_1$-$C_4$)-Alkylcarbonylamino,
($C_1$-$C_4$)-Alkylcarbonyl- ($C_1$-$C_4$)-alkylamino, Amino, Hydroxy.

4. Verfahren nach einem der Ansprüche 1 bis 3, dadurch gekennzeichnet, daß als organische Nitroverbindungen einfache Nitroaromaten wie Nitrobenzol, 2-Nitrotoluol, 4-Nitrotoluol, 4-Nitrocumol, 2-Nitro-m-xylol, 3-Nitro-o-xylol, 4-Nitro-o-xylol, 4-Nitro-m-xylol, 5-Nitro-m-xylol, Nitromesitylen,Nitroanthracen, 1-Nitronaphthalin, 2-Nitronaphthalin, 1-Nitropyren, 2-Methyl-1-nitronaphthalin, 2-Nitrofluoren, als auch komplexere Verbindungen wie 2-Nitroanisol, 3-Nitroanisol, 4-Nitroanisol, 4-Nitrobiphenyl, 2-Nitrobenzolsulfonsäureamid, 5-Nitrochinolin, 8-Nitrochinolin, 2-Nitroimidazol, 5-Nitroindazol, 6-Nitroindolin, 5-Nitroisochinolin, 4-Nitro-α-naphtholmethylether, 4-Nitrophenol, 4-Nitroanilin, Nitroresorcin, 2-Nitropyridin, 4-Nitrobenzoesäureamid, 4-Nitrobenzonitril, 4-Nitro-2,1,3-benzothiazol oder Nitroaliphaten wie Nitromethan, Nitro-tert.-butan, 1-Nitrobutan, 3-Nitro-2-butanol, Nitrocyclohexan, 1-Nitrocyclohexen, Nitrocyclopentan, Nitroethan, 2-Nitroethanol, 1-Nitrohexan, 1-Nitropropan, 2-Nitropropan, 2-Nitro-1-propanol, 2-Nitro-1-propylamin, 2-Nitro-1-ethylamin eingesetzt werden.

5. Verfahren nach einem der Ansprüche 1 bis 4, dadurch gekennzeichnet, daß als Stickstoffbase Ammoniak, Monoalkylamine, Dialkylamine, Trialkylamine, Monoalkanolamine, Dialkanolamine, Trialkanolamine, (Poly)alkylenpolyamine, N-alkylierte und gegebenenfalls an den Kohlenstoffatomen des Ringes alkylierte Piperidine und Morpholine, an dem Stickstoffatom und/oder den Kohlenstoffatomen des Ringes alkylierte Pyrrolidine, N-alkylierte und gegebenenfalls an den Kohlenstoffatomen des Ringes alkylierte Aniline und Pyridin, alkylierte Pyridine, Chinolin oder Isochinolin eingesetzt werden.

6. Verfahren nach einem der Ansprüche 1 bis 5, dadurch gekennzeichnet, daß als dreiwertige Phosphorverbindung mindestens eine Verbindung eingesetzt wird, bei der das Phosphoratom eine oder mehrere Aryl- oder Aryloxygruppen oder Alkyl- oder Alkyloxygruppen trägt, deren Kettenlänge 1-20 Kohlenstoffatome beträgt, wobei die Arylgruppen unsubstituiert oder substituiert sein können.

7. Verfahren nach einem der Ansprüche 1 bis 6, dadurch gekennzeichnet, daß das Gewichtsverhältnis eingesetzte organische Base zu eingesetzte Nitroverbindung zwischen 1 und 5 liegt.

8. Verfahren nach einem der Ansprüche 1 oder 4 bis 7, dadurch gekennzeichnet, daß die dreiwertige Phosphorverbindung in der Startreaktionsmischung in Mengen von 0,0001 - 3 Mol% bezogen auf die Nitroverbindung vorhanden ist.

9. Verfahren nach einem der Ansprüche 1 bis 8, dadurch gekennzeichnet, daß der Katalysator in Mengen von 0,001 bis 1 Gew.% bezogen auf die eingesetzte Nitroverbindung eingesetzt wird.

10. Verfahren nach einem der Ansprüche 1 bis 9, dadurch gekennzeichnet, daß es im Temperaturbereich zwischen -10°C und 100°C durchgeführt wird.

**Claims**

1. Process for preparing organic hydroxylamines, in which an organic nitro compound is partially hydrogenated in the presence of an organic base or ammonia, a trivalent phosphorus compound and a hydrogenation catalyst, characterized in that the organic base is used in an amount of greater than 10% by weight based on the organic nitro compound.

2. Process for preparing organic hydroxylamines of the formula I or II

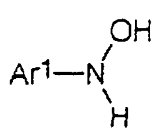

I

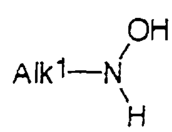

II

where $Ar^1$ is an unsubstituted or substituted isocyclic or heterocyclic aryl radical and $Alk^1$ is an unsubstituted or substituted alkyl radical, by partial hydrogenation of organic nitro compounds of the formula III or IV

$Ar^1—NO_2$ $Alk^1—NO_2$

**III** **IV**

where $Ar^1$ and $Alk^1$ are as defined above, in the presence of a hydrogenation catalyst comprising at least one element of transition group VII or VIII of the Periodic Table and at least one nitrogen base and at least one trivalent phosphorus compound, characterized in that the nitrogen base and the nitro compound are used in a weight ratio of organic [sic] base to nitro compound of greater than 0.1 and the trivalent phosphorus compound is used in an amount of 0.0001-3 mol% based on the nitro compound.

**3.** Process according to Claim 2, characterized in that $Ar^1$ in the formulae I and III is an isocyclic or heterocyclic aryl radical which has one or two rings and may be substituted by one or more substituents $R^1$, where $R^1$ may be identical or different and each have one of the following meanings:
halogen atom, $C_1$-$C_4$-alkyl, halogen-($C_1$-$C_4$)-alkyl, halogen($C_1$-$C_4$)-alkoxy, $C_1$-$C_4$-alkoxy, $C_1$-$C_4$-alkylthio, ($C_1$-$C_4$)-alkylcarbonyl, ($C_1$-$C_4$)-alkoxycarbonyl, ($C_1$-$C_4$)-alkylaminocarbonyl, ($C_1$-$C_4$)-dialkylaminocarbonyl, ($C_1$-$C_4$)-dialkylamino, ($C_1$-$C_4$)-alkylamino, ($C_1$-$C_4$)-alkylcarbonylamino, ($C_1$-$C_4$)-alkylcarbonyl-($C_1$-$C_4$)-alkylamino, cyano, nitro, amino, hydroxy, carboxy, ester or salt of the carboxyl group, sulphono, ester or salt of the sulphono group or A-B, where A is -O-, -S-, -O-$CH_2$-, -$CH_2$-O-, -S-$CH_2$-, -$CH_2$-S-,-$CH_2$-O-CO-, -$CH_2$-N($R^2$)-, -CH=CH-, -CH=N-O- or a single bond, and
$R^2$ is $C_1$-$C_4$-alkyl, $C_2$-$C_4$-alkenyl or hydrogen
and
B is phenyl, naphthyl, pyridinyl, pyrazinyl, pyrimidinyl, pyridazinyl, pyrazolyl, imidazolyl, oxazolyl, isoxazolyl, thiazolyl, isothiazolyl, furanyl, thienyl, pyrrolyl or $C_3$-$C_7$-cycloalkyl,
where B may be substituted by 1-3 substituents $R^3$ and $R^3$ is halogen, $C_1$-$C_4$-alkyl, halo-($C_1$-$C_4$)-alkyl, halo-($C_1$-$C_4$)-alkoxy, $C_1$-$C_4$-alkoxy, $C_1$-$C_4$-alkylthio, ($C_1$-$C_4$)-alkylcarbonyl, ($C_1$-$C_4$)-alkoxycarbonyl, ($C_1$-$C_4$)-alkylaminocarbonyl, ($C_1$-$C_4$)-dialkylaminocarbonyl, ($C_1$-$C_4$)-alkylcarbonylamino, ($C_1$-$C_4$)-alkylcarbonyl-($C_1$-$C_4$)-alkylamino or cyano, and $Alk^1$ in the formulae II and IV is a linear or branched or cyclic alkyl radical having 1-24 carbon atoms, where the alkyl radical may be saturated or unsaturated and is unsubstituted or substituted by one or more substituents $R^4$, where $R^4$ may be identical or different and each have one of the following meanings:
$C_1$-$C_4$-alkoxy, $C_1$-$C_4$-alkylthio, ($C_1$-$C_4$)-alkylcarbonyl, ($C_1$-$C_4$)-alkoxycarbonyl, ($C_1$-$C_4$)-alkylaminocarbonyl, ($C_1$-$C_4$)-dialkylaminocarbonyl, ($C_1$-$C_4$)-dialkylamino, ($C_1$-$C_4$)-alkylamino, ($C_1$-$C_4$)-alkylcarbonylamino, ($C_1$-$C_4$)-alkylcarbonyl($C_1$-$C_4$)-alkylamino, amino, hydroxy.

**4.** Process according to any of Claims 1 to 3, characterized in that the organic nitro compounds used are simple nitroaromatics such as nitrobenzene, 2-nitrotoluene, 4-nitrotoluene, 4-nitrocumene, 2-nitro-m-xylene, 3-nitro-o-xylene, 4-nitro-o-xylene, 4-nitro-m-xylene, 5-nitro-m-xylene, nitromesitylene, nitroanthracene, 1-nitronaphthalene, 2-nitronaphthalene, 1-nitropyrene, 2-methyl-1-nitronaphthalene and 2-nitrofluorene and also more complex compounds such as 2-nitroanisole, 3-nitroanisole, 4-nitroanisole, 4-nitrobiphenyl, 2-nitrobenzenesulphonamide, 5-nitroquinoline, 8-nitroquinoline, 2-nitroimidazole, 5-nitroindazole, 6-nitroindoline, 5-nitroisoquinoline, 4-nitro-$\alpha$-naphthol methyl ether, 4-nitrophenol, 4-nitroaniline, nitroresorcinol, 2-nitropyridine, 4-nitrobenzamide, 4-nitrobenzonitrile and 4-nitro-2,1,3-benzothiazole, or nitroaliphatics such as nitromethane, nitro-tert-butane, 1-nitrobutane, 3-nitro-2-butanol, nitrocyclohexane, 1-nitrocyclohexene, nitrocyclopentane, nitroethane, 2-nitroethanol, 1-nitrohexane, 1-nitropropane, 2-nitropropane, 2-nitro-1-propanol, 2-nitro-1-propylamine and 2-nitro-1-ethylamine.

**5.** Process according to any of Claims 1 to 4, characterized in that the nitrogen base used is ammonia, a monoalkylamine, dialkylamine, trialkylamine, monoalkanolamine, dialkanolamine, trialkanolamine or (poly) alkylenepolyamine, an N-alkylated piperidine or morpholine which may also be alkylated on the carbon atoms of the ring, a pyrrolidine alkylated on the nitrogen atom and/or the carbon atoms of the ring, an N-alkylated aniline which may also be alkylated on the carbon atoms of the ring, or pyridine an alkylated pyridine, quinoline or isoquinoline.

**6.** Process according to any of Claims 1 to 5, characterized in that the trivalent phosphorus compound used is at least one compound in which the phosphorus atom bears one or more aryl or aryloxy groups or alkyl or alkoxy groups whose chain length is 1-20 carbon atoms, where the aryl groups may be unsubstituted or substituted.

**7.** Process according to any of Claims 1 to 6, characterized in that the weight ratio of organic base used to nitro

compound used is in the range from 1 to 5.

8. Process according to any of Claims 1 and 4 to 7, characterized in that the trivalent phosphorus compound is present in the initial reaction mixture in an amount of 0.0001-3 mol% based on the nitro compound.

9. Process according to any of Claims 1 to 8, characterized in that the catalyst is used in an amount of from 0.001 to 1% by weight based on the nitro compound used.

10. Process according to any of Claims 1 to 9, characterized in that it is carried out in the temperature range from -10°C to 100°C.

**Revendications**

1. Procédé pour la préparation d'hydroxylamines organiques dans lequel on hydrogène partiellement un composé organique nitré en présence d'une base organique ou d'ammoniac, d'un composé phosphoré trivalent et d'un catalyseur d'hydrogénation, caractérisé en ce que la base organique est utilisée en des quantités supérieures à 10% en poids par rapport au composé organique nitré.

2. Procédé pour la préparation d'hydroxylamines organiques de formules générales I ou II

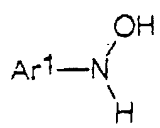

I

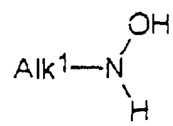

II

dans lesquelles $Ar^1$ représente un radical aryle non substitué ou substitué, isocyclique ou hétérocyclique, et $Alk^1$ est un radical alkyle non substitué ou substitué, par hydrogénation partielle de composés organiques nitrés de formules générales III ou IV

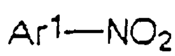

III

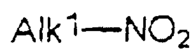

IV

dans lesquelles $Ar^1$ et $Alk^1$ ont les significations mentionnées, en présence d'un catalyseur d'hydrogénation contenant au moins un élément du 7ème ou du 8ème groupe secondaire du système périodique et d'au moins une base azotée et d'au moins un composé phosphoré trivalent, caractérisé en ce que la base azotée et le composé nitré sont utilisés dans un rapport pondéral de base organique à composé nitré supérieur à 0,1 et le composé phosphoré trivalent en une quantité de 0,0001 à 3% en mole par rapport au composé nitré.

3. Procédé selon la revendication 2, caractérisé en ce que $Ar^1$ dans les formules I et III représente un radical aryle isocyclique ou hétérocyclique à un ou deux noyaux, qui peut être monosubstitué ou polysubstitué par $R^1$, $R^1$ étant identique ou différent et présentant une des significations suivantes :
atome d'halogène, alkyle en $C_1$ à $C_4$, halogène(alkyle en $C_1$ à $C_4$), halogène(alcoxy en $C_1$ à $C_4$), alcoxy en $C_1$ à $C_4$, (alkyle en $C_1$ à $C_4$)thio, (alkyle en $C_1$ à $C_4$)carbonyle, (alcoxy en $C_1$ à $C_4$)carbonyle, (alkyle en $C_1$ à $C_4$)aminocarbonyle, (dialkyle en $C_1$ à $C_4$)aminocarbonyle, (dialkyle en $C_1$ à $C_4$)amino, (alkyle en $C_1$ à $C_4$)amino, (alkyle en $C_1$ à $C_4$)carbonylamino, (alkyle en $C_1$ à $C_4$)carbonyl(alkyle en $C_1$ à $C_4$)amino, cyano, nitro, amino, hydroxy, carboxy, ester ou sel du radical carboxy, sulfono, ester ou sel du radical sulfono ou A-B, A signifiant -O-, -S-, -O-$CH_2$-, -$CH_2$-O-, -S-$CH_2$-, -$CH_2$-S-, -$CH_2$-O-CO-, -$CH_2$-N($R^2$)-, -CH=CH-, -CH=N-O- ou une simple liaison et
$R^2$ signifiant un alkyle en $C_1$ à $C_4$, un alcényle en $C_2$ à $C_4$ ou un hydrogène et
B signifiant un phényle, un naphtyle, un pyridinyle, un pyrazinyle, un pyrimidinyle, un pyridazinyle, un pyrazolyle,

un imidazolyle, un oxazolyle, un isoxazolyle, un thiazolyle, un isothiazolyle, un furannyle, un thiényle, un pyrrolyle ou un cycloalkyle en $C_3$ à $C_7$,
B pouvant être substitué par 1 à 3 substituants $R^3$ et $R^3$ signifiant un halogène, un alkyle en $C_1$ à $C_4$, un halo-(alkyle en $C_1$ à $C_4$), un halo(alcoxy en $C_1$ à $C_4$), un alcoxy en $C_1$ à $C_4$, un (alkyle en $C_1$ à $C_4$)thio, un (alkyle en $C_1$ à $C_4$)carbonyle, un (alcoxy en $C_1$ à $C_4$)carbonyle, un (alkyle en $C_1$ à $C_4$)aminocarbonyle, un (dialkyle en $C_1$ à $C_4$) aminocarbonyle, un (alkyle en $C_1$ à $C_4$)carbonylamino, un (alkyle en $C_1$ à $C_4$)carbonyl(alkyle en $C_1$ à $C_4$)amino ou un cyano et
$Alk^1$ dans les formules II et IV représente un radical alkyle linéaire ou ramifié ou cyclique comprenant 1 à 24 atomes de carbone, le radical alkyle pouvant être saturé ou insaturé et étant non substitué ou monosubstitué ou polysubstitué par les substituants $R^4$, $R^4$ pouvant-être identique ou différent et présentant une des significations suivantes : alcoxy en $C_1$ à $C_4$, (alkyle en $C_1$ à $C_4$)thio, (alkyle en $C_1$ à $C_4$)carbonyle, (alcoxy en $C_1$ à $C_4$)carbonyle, (alkyle en $C_1$ à $C_4$) aminocarbonyle, (dialkyle en $C_1$ à $C_4$)aminocarbonyle, (dialkyle en $C_1$ à $C_4$)amino, (alkyle en $C_1$ à $C_4$) amino, (alkyle en $C_1$ à $C_4$)carbonylamino, (alkyle en $C_1$ à $C_4$)carbonyl(alkyle en $C_1$ à $C_4$)amino, amino, hydroxy.

**4.** Procédé selon l'une quelconque des revendications 1 à 3, caractérisé en ce qu'on utilise comme composés organiques nitrés des nitroaromates simples, tels que le nitrobenzène, le 2-nitrotoluène, le 4-nitrotoluène, le 4-nitrocumène, le 2-nitro-m-xylène, le 3-nitro-o-xylène, le 4-nitro-o-xylène, le 4-nitro-m-xylène, le 5-nitro-m-xylène, le nitromésitylène, le nitroanthracène, le 1-nitronaphtalène, le 2-nitronaphtalène, le 1-nitropyrène, le 2-méthyl-1-nitronaphtalène, le 2-nitrofluorène, ainsi que des composés plus complexes tels que le 2-nitroanisol, le 3-nitroanisol, le 4-nitroanisol, le 4-nitrobiphényle, l'amide de l'acide 2-nitrobenzènesulfonique, la 5-nitroquinoléine, la 8-nitroquinoléine, le 2-nitroimidazol, le 5-nitroindazol, le 6-nitroindoline, la 5-nitroisoquinoléine, le 4-nitro-α-naphtolméthyléther, le 4-nitrophénol, la 4-nitroaniline, la nitrorésorcine, la 2-nitropyridine, l'amide de l'acide 4-nitrobenzoïque, le 4-nitrobenzènenitrile, le 4-nitro-2,1,3-benzothiazol ou des nitroaliphates tels que le nitrométhane, le nitro-tert-butane, le 1-nitrobutane, le 3-nitro-2-butanol, le nitrocyclohexane, le 1-nitrocyclohexène, le nitrocyclopentane, le nitroéthane, le 2-nitroéthanol, le 1-nitrohexane, le 1-nitropropane, le 2-nitropropane, le 2-nitro-1-propanol, la 2-nitro-1-propylamine, la 2-nitro-1-éthylamine.

**5.** Procédé selon l'une quelconque des revendications 1 à 4, caractérisé en ce qu'on utilise comme base azotée de l'ammoniac, des monoalkylamines, des dialkylamines, des trialkylamines, des monoalcanolamines, des dialcanolamines, des trialcanolamines, des (poly)alkylènepolyamines, des pipéridines et des morpholines N-alkylées et le cas échéant alkylées sur les atomes de carbone du noyau, des pyrrolidines alkylées sur l'atome d'azote et/ou les atomes de carbone du noyau, des anilines et de la pyridine N-alkylées et le cas échéant alkylées sur les atomes de carbone du noyau, des pyridines alkylées, de la quinoléine ou de l'isoquinoléine.

**6.** Procédé selon l'une quelconque des revendications 1 à 5, caractérisé en ce qu'on utilise comme composé phosphoré trivalent au moins un composé dans lequel l'atome de phosphore porte un ou plusieurs groupements aryle ou aryloxy ou un ou plusieurs groupements alkyle ou alcoxy, dont la longueur de chaîne est de 1 à 20 atomes de carbone, les groupements aryle pouvant être non substitués ou substitués.

**7.** Procédé selon l'une quelconque des revendications 1 à 6, caractérisé en ce que le rapport pondéral base organique utilisée à composé nitré utilisé est situé entre 1 et 5.

**8.** Procédé selon l'une quelconque des revendications 1 ou 4 à 7, caractérisé en ce que le composé phosphoré trivalent dans le mélange réactionnel de départ est présent en des quantités de 0,0001 à 3% en mole par rapport au composé nitré.

**9.** Procédé selon l'une quelconque des revendications 1 à 8, caractérisé en ce que le catalyseur est utilisé en des quantités de 0,001 à 1% en poids par rapport au composé nitré utilisé.

**10.** Procédé selon l'une quelconque des revendications 1 à 9, caractérisé en ce qu'il est réalisé dans une plage de températures entre -10°C et 100°C.